# EUROPEAN PATENT SPECIFICATION

(11) **EP 3 976 041 B1**
(45) Date of publication and mention of the grant of the patent: **12.11.2025**
(21) Application number: 20730699.4
(22) Date of filing: 29.05.2020
(51) Int. Cl.: A61P 19/02, A61K 31/506

(54) **TALTIRELIN USE TO TREAT OSTEOARTRITIS**
ANWENDUNG VON TALTIRELIN ZUR BEHANDLUNG VON ARTHROSE
UTILISATION DE TALITRÉLINE POUR TRAITER L'ARTHROSE

(30) Priority: 29.05.2019 GB 201907591
(43) Date of publication of application: 06.04.2022
(73) Proprietor: Eolas Research Limited, Edinburgh EH9 3AL (GB)
(72) Inventor: FORBES, Iain, Edinburgh EH9 3AL (GB); FORBES, William, Edinburgh EH9 3AL (GB)
(74) Representative: Jacob, Reuben Ellis
(86) International application number: PCT/GB2020/051315
(87) International publication number: WO 2020/240207

(56) References cited:
- EP-A1- 3 520 795
- WO-A2-2006/096829
- CN-A- 104 458 929
- US-A- 4 665 056
- US-A1- 2014 271 730
- US-A1- 2014 271 809
- MA ET AL: "The Roles of FoxO Transcription Factors in Regulationof Bone Cells Function", INT.J.MOL.SCI. 2020,21,692 (PP.1-22), 21 January 2020 (2020-01-21)
- MATSUZAKI ET AL: "FoxO transcription factors influence cartilage maturation, homeostasis and osteoarthritis pathogenesis by modulating autophagy and proteoglycan 4", SCI.TRANSL.MED. 2018,10(428), (PP.1-25), 14 February 2018 (2018-02-14)
- CHUNLEI NIE ET AL: "Thyrotropin-releasing hormone and its analogs accelerate wound healing", JOURNAL OF SURGICAL RESEARCH., vol. 189, no. 2, 1 June 2014 (2014-06-01), US, pages 359 - 365, XP055710179, ISSN: 0022-4804, DOI: 10.1016/j.jss.2014.03.004
- ZENICHIRO KATO ET AL: "Oral Administration of the Thyrotropin-Releasing Hormone (TRH) Analogue, Taltireline Hydrate, in Spinal Muscular Atrophy", JOURNAL OF CHILD NEUROLOGY., vol. 24, no. 8, 1 August 2009 (2009-08-01), CA, pages 1010 - 1012, XP055710024, ISSN: 0883-0738, DOI: 10.1177/0883073809333535
- KEI ETO ET AL: "Taltirelin, a thyrotropin-releasing hormone analog, alleviates mechanical allodynia through activation of descending monoaminergic neurons in persistent inflammatory pain", BRAIN RESEARCH, ELSEVIER, AMSTERDAM, NL, vol. 1414, 30 July 2011 (2011-07-30), pages 50 - 57, XP028293574, ISSN: 0006-8993, [retrieved on 20110806], DOI: 10.1016/J.BRAINRES.2011.07.065
- WALTER PIERPAOLI: "Aging-reversing properties of thyrotropin-releasing hormone", CURRENT AGING SCIENCE, vol. 6, 1 January 2013 (2013-01-01), pages 92 - 98, XP055710029
- R SCHRAGE ET AL: "Superagonism at G protein-coupled receptors and beyond : GPCR superagonism", BRITISH JOURNAL OF PHARMACOLOGY, vol. 173, no. 20, 1 October 2016 (2016-10-01), UK, pages 3018 - 3027, XP055711164, ISSN: 0007-1188, DOI: 10.1111/bph.13278
- HIDETOSHI ASAI ET AL: "Reversal of hemorrhagic shock in rats using the metabolically stable thyrotropin-releasing hormone analog taltirelin hydrate", JOURNAL OF RECEPTOR AND SIGNAL TRANSDUCTION RESEARCH., vol. 31, no. 6, 31 December 2011 (2011-12-31), US, pages 416 - 422, XP055711168, ISSN: 1079-9893, DOI: 10.3109/10799893.2011.625427
- AHMED ESSAGHIR ET AL: "The Transcription of FOXO Genes Is Stimulated by FOXO3 and Repressed by Growth Factors", JOURNAL OF BIOLOGICAL CHEMISTRY, vol. 284, no. 16, 17 April 2009 (2009-04-17), US, pages 10334 - 10342, XP055710939, ISSN: 0021-9258, DOI: 10.1074/jbc.M808848200
- CARSTEN SKURK ET AL: "The Akt-regulated Forkhead Transcription Factor FOXO3a Controls Endothelial Cell Viability through Modulation of the Caspase-8 Inhibitor FLIP", JOURNAL OF BIOLOGICAL CHEMISTRY, vol. 279, no. 2, 8 October 2003 (2003-10-08), US, pages 1513 - 1525, XP055711243, ISSN: 0021-9258, DOI: 10.1074/jbc.M304736200
- TAKAAKI YAMAMURA ET AL: "ONCOGENICITY STUDIES OF TALTIRELIN TETRAHYDRATE (TA-0910) BY ORAL (GAVAGE) ADMINISTRATION IN CD-1 MICE AND CD RATS", JOURNAL OF TOXICOLOGICAL SCIENCES., vol. 22, no. SupplementII, 1 January 1997 (1997-01-01), JP, pages 419 - 430, XP055711258, ISSN: 0388-1350, DOI: 10.2131/jts.22.SupplementII_419

## Description

The invention relates to the use of a composition for the prevention, amelioration and/ or treatment of conditions associated with ageing and senescence, the process of ageing, premature ageing and susceptibility to disease as a result of ageing and/ or senescence, and/ or inflammatory and/ or autoimmune conditions.

Thyrotropin-releasing hormone (TRH) is a small peptide which is found in the hypothalamus of the brain and emanates from a group of neurones. The native peptide is highly bio-active and controls the release of thyroglobulin, which in turn is responsible for the induction of thyroid hormones. TRH and associated analogues are useful for the treatment or prophylaxis of central nervous disorders (e.g. as described in JPS 62234029 and EP 0168042).

Taltirelin (also known as Ceredist) is a thyrotropin-releasing hormone analogue which mimics the physiological actions of TRH but has a much longer half life and duration of effects. Taltirelin has been used in the treatment of neurological conditions. The following publication may be useful to understand present invention: Ma et al: Int.J.Mol.Sci. 2020,21,692 (pp.1-22) and D17 Matsuzaki et al: Sci.Transl.Med. 2018,10(428), (pp.1-25).

Ageing in mammals involves the progressive loss of function in cells and/or tissues involving a number of interconnected biological processes including, cell senescence, inflammaging/ immunosenescence, autophagy, reduction in the stem cell pool, reduction in stem cell regeneration and mitochondrial dysfunction. This age-related loss of function is influenced by both genetics and environmental factors and leads to a wide range of degenerative conditions, diseases and pathologies. Ageing is the greatest risk factor for many of the most common morbidities, including cancer, cardiovascular and neurodegenerative diseases as well as a reduction in immunity resulting in reduced T-Cell and B-Cell production and diminished function of lymphocytes leading to a diminished response to viral, bacterial and parasitic infections.

The term senescence, including cellular senescence, involves perceptible changes in cells such as telomere attrition, double-strand DNA breaks, persistent DNA damage response, oxidative stress, oncogene activation and an increase in the levels of cell-cycle arrest proteins p16 INK4a and p21 all leading to an exit from the normal cell-cycle, a process termed senescence associated growth arrest. These senescent cells, with age, tend to accumulate and become increasingly harmful to surrounding cells and tissues and in addition become increasingly likely to escape from the state of growth arrest to become cancerous. Further, these accumulating senescent cells result in an increasing loss in the physiological functioning in a tissue and the prevention of new healthy cells from replacing them. Senescent cells in the long term contribute further to ageing effects by secreting pro-inflammatory and growth factors. The process of senescence may be natural, premature, induced or accelerated. Induced or accelerated senescence has been found to be involved in some diseases, and in transplanted cells, tissues and organs. Accelerated senescence has been observed in diseased organs such as the kidney.

The term inflammaging involves chronic low grade inflammatory processes, in the absence of infection, linked to ageing *via* immunosenescence. Senescent cells develop a so-called senescence-associated secretory phenotype (SASP), which involves the secretion of pro-inflammatory factors which are thought to contribute to inflammaging. This SASP is a major risk factor for age-related diseases.

The term autophagy, which includes mitophagy, is involved the intracellular degradation process in healthy cells that helps maintain cellular homeostasis by degrading and recycling dysfunctional and excess cellular components. Many physiological functions are regulated by autophagy, including senescence, longevity tissue remodelling and cell survival during stress. Reduced autophagy has been found to be involved in the pathogenesis and/ or progression of age-related diseases such as inflammatory and autoimmune diseases including osteoarthritis, lupus erythematosus, Crohn's disease, and inflammatory bowel disease. In addition, reduced autophagy has also been associated with cancers, metabolic diseases, fibrotic diseases including for example idiopathic pulmonary disease, heart disease, as well as neurodegenerative diseases such as Parkinson's disease and Huntington's disease. Reduced autophagy has also been linked to psychiatric disorders such as schizophrenia and bipolar disorder.

The terms stem cell pool and stem cell regeneration involve the population of cells that can replenish tissue with a range of newly differentiated cell types and the regeneration of a variety of cell types from so-called stem cells, thus allowing the repair and maintenance of healthy tissue of tissues. Ageing is characterised by a reduction in the capability of stem cells to regenerate cells in tissues.

The present invention seeks to provide a composition for use in preventing, ameliorating and/ or treating conditions which involve the processes of ageing, including senescence and/ or inflammation and/ or autophagy and/ or reduction in the stem cell pool and/ or stem cell regenerative capability including lymphoid biased stem cells.

According to the invention, there is provided use of taltirelin as defined in the appended claims.

The taltirelin that is used in the present invention comprises anti-senescence, and/or anti-inflammatory and/ or anti-proliferative and/ or pro-autophagic and/ or pro- stem cell regenerative properties and/ or stem cell pool maintenance properties. Typically, the use of taltirelin in accordance with the present invention is involved in the induction of FOXO gene expression, which expression decreases with age and which increased expression advantageously reduces the effects of ageing and increases longevity, increases immune cell homeostasis, differentiation, reactivation and self-renewal, controls the trajectory of T-Cell dependent immune response, increases survival of CD8+ memory cells and antiviral CD8+ cells and in addition advantageously activates and regulates key leukocytes, including the formation of T-regulatory cells and B-lymphocytes needed to maintain homeostasis and respond to infection in mucosal tissues including oral mucosal tissues.

Advantageously, the taltirelin may be used in human therapeutics and/ or in veterinary practice, and is typically administered to a mammalian subject.

Advantageously, the taltirelin may be administered with a high degree of safety.

The term senescence may relate to natural, premature, induced or accelerated senescence.

The invention will be further described by way of example and with reference to the following figures, in which:
Figure 1 is a graph illustrating the increased expression of FOXO1 and FOXO3 genes in fibroblasts by the administration of taltirelin; and
Figure 2 is a graph illustrating the increased expression of EFNB1, NFE2L2, SERPINE1 and SIRT1 genes in fibroblasts by administration of taltirelin.

With reference to the figures, there is provided use of taltirelin (N-[[(4S)-Hexahydro-1-methyl-2,6-dioxo-4-pyrimidinyl]carbonyl]-L-histidyl-L-prolinamide) having the structure: 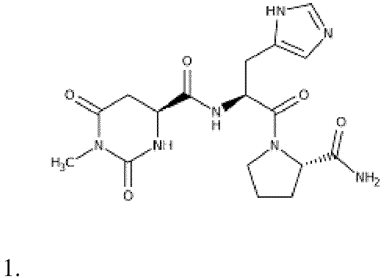 for use in the treatment of a condition associated with cell or tissue senescence or inflammation, wherein the condition is osteoarthritis.

In one example, the taltirelin may be administered with at least one acceptable carrier and/ or may be carried within a vesicle, micelle, liposome, nanoparticle or other suitable vehicle.

The taltirelin may be used at a dose of around 5 to 15 mg per kilogram of body weight per day, especially at a dose of 10 mg per kilogram of body weight per day. Typically, administration may be via an oral or parenteral (e.g. via the intravenous, intramuscular or subcutaneous) route. Typically, the therapeutic dose of the taltirelin depends on the route of administration; the age, weight and condition of patients; and the particular disease to be treated.

The taltirelin of the present invention comprises anti-senescence, and/or anti-inflammatory and/ or anti-proliferative and/ or pro-autophagic and/ or pro- stem cell regenerative properties and/ or stem cell pool maintenance properties. The use of taltirelin of the present invention is involved in the induction of FOXO gene expression, which expression advantageously reduces the effects of ageing and increases longevity.

### Description of FOXO gene effects:

The FOXO transcription factors are encoded by the FOXO genes and in mammals comprise FOXO1, FOXO3, FOXO4 and FOXO6.

FOXO1 is involved in the regulation of proliferation, oxidative stress resistance, metabolism, inflammation, ageing, activation of autophagy and apoptosis. The FOXO1 gene regulates glucose levels in gluconeogenesis and is a negative regulator of adipogenesis and also regulates glycogenolysis. FOXO1 typically plays a role as a tumour suppressor and may induce apoptosis is some cancers and also suppress the metastasis of some cancer cell types. The transcription factor of the FOXO1 gene (Foxo1) is decreased in certain drug resistant cancer cells. The transcription factor Foxo1 also regulates osteoblast differentiation and skeletogenesis and therefore is of importance in disease of bone. Furthermore, FOXO1 is a potent inhibitor of fibrogenic signaling and extra-cellular matrix deposition which is crucial in the pathogenesis and progression of fibrosis. Foxo1 transcription factor represses the Epithelial - Mesenchymal transition (EMT) which is involved in the pathogenesis of fibrosis and in (EMT) cancers. Foxo1, through its induction of autophagy may influence adult neurogenesis by overcoming aberrant dendritic morphology, particularly in the subgranular zone of the hippocampal nucleus and also in the subventricular zone - areas involved in memory, learning and cognitive function.

FOXO3 is involved the regulation of proliferation, oxidative stress resistance, metabolism, cellular differentiation, inflammation, ageing, proteostasis, stem cell pool maintenance, activation of autophagy and apoptosis.

Genetic polymorphisms of FOXO3 have shown consistent associations with human longevity and in *in-vivo* models.

FOXO3 enhances cell self-renewal via the activation of Notch signalling and has been shown to enhance pluripotency in some stem cells. FOXO3 is crucial for the regulation of adult stem cell homeostasis and is the primary driver of the maintenance of the stem cell pool in mammals. FOXO3 has also been shown to promote the self re-renewal of stem cells during muscle regeneration. Foxo3, through its induction of autophagy may influence adult neurogenesis by overcoming aberrant dendritic morphology, particularly in the subgranular zone of the hippocampal nucleus and also in the subventricular zone, i.e. areas involved in memory, learning and cognitive function.

The transcription factor of the FOXO3 gene (Foxo3) is decreased in certain drug resistant cancer cells.

Furthermore, FOXO3 is a potent inhibitor of fibrogenic signalling and extra-cellular matrix deposition that are crucial in the pathogenesis and progression of fibrosis, including liver fibrosis, cystic fibrosis and that of the lung, heart and kidney. Foxo3 transcription factor represses the Fibroblast Myofibroblast transition (FMT) which is involved in the pathogenesis of fibrosis. FOXO3 may also have beneficial effects on glucose metabolism through more favourable insulin sensitivity. FOXO3 controls cytokine production and lowers inflammation.

FOXO 3 transcription factor represses the Epithelial Mesenchymal transition (EMT) which is involved in the pathogenesis of fibrosis and in (EMT) cancers.

### Gene Expression

Experiments have shown that taltirelin modulates the gene expression in fibroblasts of a variety of genes involved in processes underlying ageing. Principally, the compound increases expression of the FOXO genes, notably FOXO1 and FOXO3.

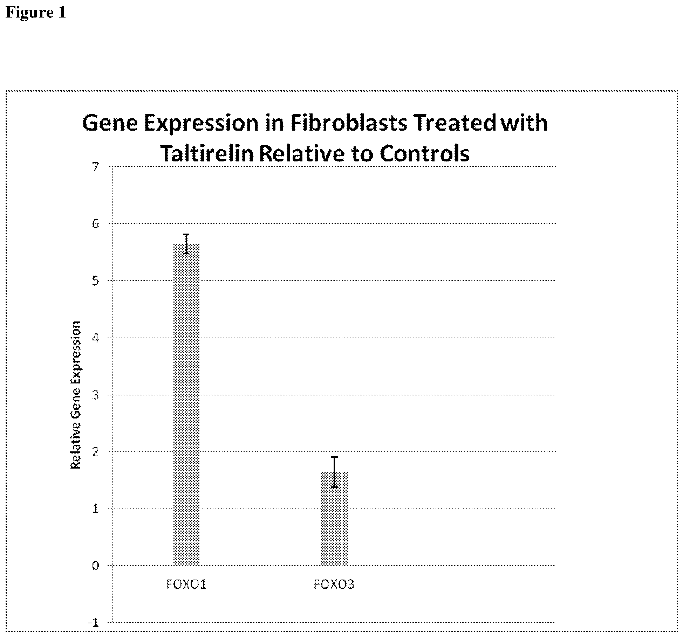

Figure 1 illustrates the increased expression of FOXO1 and FOXO3 genes that occurs in fibroblasts by administration of taltirelin. From the results, it can be seen that the administration of taltirelin in older cells (passage 20) led to a large increase in the expression of FOXO1 (>5X) and FOXO3 (>1.5X) compared to untreated controls. The results were statistically significant with P<0.01.

### COVID-19 caused by SARS-CoV-2 (outside the scope of the invention)

In certain people the immune response may be weak. The weakening of the immune response often occurs as people get older. In such people it would be advantageous to strengthen the immune response, for example, by expanding the T cell population.

FOXO 1 expands the population of T cells and B cells within an individual. T cells are produced by the thymus which decreases in size with age. The upregulation of FOXO 1 (as shown in Figure 1) has the effect of boosting the immune response, thus increasing the T cell population within an individual.

FOXO 1 also upregulates and enhances the survival of the CD8+ population that is involved in immune memory. For example, after an individual has had a viral attack their immune memory will result in an immediate response to a subsequent viral infection.

FOXO1 acts as the control point for a programme of gene expression that allows T-cells to undergo serial reactivation and renewal involved in long term immunity.

In addition, FOXO 3 upregulates and activate the interferon group (i.e. interferon α, interferon β and interferon γ). Interferon γ inhibits IL-13. IL-13 activates a receptor (TMPRSS2) on a host cell that severe acute respiratory syndrome coronavirus 2 (SARS-CoV-2) uses to enter the cell. FOXO 3 has been shown to inhibit the mechanism by which SARS-CoV-2 enters the host cell, via the TMPRSS2 receptor.

Advantageously, the composition of the present invention acts to block the activation of the TMPRSS2 receptor on host cells before and during a viral infection and to prevent cleavage of the spike protein of a coronavirus, particularly of SARS-CoV-2.

T-cells, including CD8+ T-cells are decreased markedly in patients with SARS-CoV-2 infection and there is functional exhaustion of cytotoxic lymphocytes associated with SARS-CoV-2 infection.

In addition, in adaptive immunity, FOXO1 increases expression of receptors that control T-cell trafficking to secondary lymphoid tissues and that the survival and homeostasis of T-cells are influenced by IL-7 and that FOXO1 controls T-cell tolerance and naive T-Cell homeostasis through induction of IL-7R expression.

Further, FOXO 1 also regulates the homing of peripheral B cells through upregulation of L-selectin and regulates class-switch recombination in B-cells.

In addition, FOXO1 and FOXO3 are involved in oxidative stress resistance and that oxidative stress is a key player in viral infection including SARS-CoV-2 infection.

Thus, a composition comprising taltirelin or an analogue thereof may be used for the prevention, amelioration and/ or treatment of a condition caused by a virus such as a coronavirus, such as severe acute respiratory syndrome coronavirus 2 (SARS-CoV-2) via its effects of upregulating the expression of FOXO 1 and FOXO 3.

### The Sirtuins

SIRT1 (Sirt 1) expression was also increased significantly by taltirelin. Sirt 1 inhibits adipogenesis. Sirtion 1 expression induces autophagy. A high level of expression of SIRT1 is thought to be protective in many age-related diseases, including cardiovascular diseases, metabolic syndrome, neurodegeneration and cancer.

### The Ephrins

EFNB1 was significantly increased in fibroblasts by taltirelin. EFNB1 contributes to the suppression of the adipose inflammatory response and in obesity reduction on adipose EFNB1 may contribute to adipose tissue inflammation.

### SERPINE1 (PAI-1)

SERPINE1 expression is significantly inhibited in fibroblasts by taltirelin. Obese patients with the age-related disease, metabolic syndrome and type-2 diabetes show an increase in levels of the product of this gene (PAI-1). Increased expression of this gene is also associated with enhanced radioresistance and tumour aggressiveness in non-small cell lung cancer cells. Furthermore, the product of the gene SERPINE1 (PAI-1) is the main inhibitor of the plasminogen activator system, which blocks fibrinolysis and promotes ECM accumulation in tissues therefore promoting fibrosis.

### NFE2L2 (Nrf2)

NFE2L2 expression is significantly increased in fibroblasts by taltirelin. The product of the gene NFE2L2 (Nrf2) inhibits EMT and fibrosis. NFE2L2 (Nrf2) expression decreases with age. NFE2L2 (Nrf2) expression is a critical regulator of the innate immune response. NFE2L2 (Nrf2) has a crucial role in viral entry and replication in tissues of the nasal passages.

NFE2L2 (Nrf2) controls cellular redox balance and the expression of a wide array of genes involved in immunity and inflammation, including antiviral actions. Nrf2 activity declines with age, making the elderly more susceptible to oxidative stress-mediated diseases, which include type 2 diabetes, chronic inflammation, and viral infections.

NFE2L2 (Nrf2) may also control viral susceptibility and replication.

NFE2L2 (Nrf2) activating compounds can downregulate ACE2 and TMPRSS2mRNA expression. ACE2 is a surface receptor and TMPRSS2 activates the spike protein for SARS-Cov-2 entry into host cells.

Further, NFE2L2 (Nrf2) is a master antioxidant transcription factor and is abundant in the lungs, drives diverse cellular defence pathways to counteract detrimental stimuli that are involved in the pathogenesis of these pulmonary disorders, including oxidative stress, inflammation, apoptosis, and carcinogenesis.

Thus, a composition comprising taltirelin or an analogue thereof may be used for the prevention, amelioration and/ or treatment of a condition caused by a virus such as a coronavirus, such as severe acute respiratory syndrome coronavirus 2 (SARS-CoV-2) via its effects on the innate immune response and on viral entry to the body and subsequent replication in tissues at the point of entry (outside the scope of the invention).

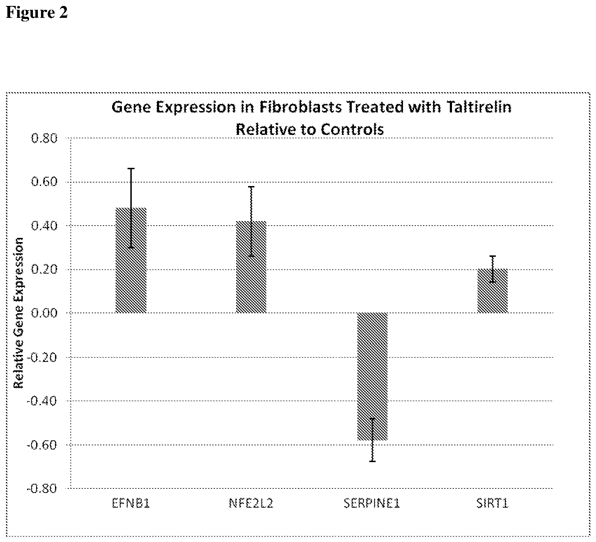

Figure 2 illustrates the increased expression of EFNB1, NFE2L2, SERPINE1 and SIRT1 genes in fibroblasts that occurs by administration of taltirelin. From the results, it can be seen that the administration of taltirelin in older cells (passage 20) led to a statistically significant (P<0.01) increase in the expression of EFNB1, NFE2L2 and SIRT1 genes and a statistically significant (P<0.01) decrease in the expression of the SERPINE1 gene compared to untreated controls.

## Claims

1. Taltirelin (N-[[(4S)-Hexahydro-1-methyl-2,6-dioxo-4-pyrimidinyl]carbonyl]-L-histidyl-L-prolinamide) having the structure: 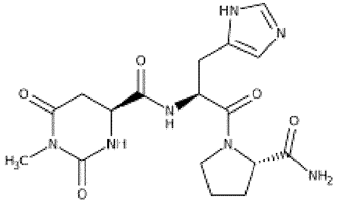 for use in the treatment of a condition associated with cell or tissue senescence or inflammation, wherein the condition is osteoarthritis.

## Patentansprüche

1. Taltirelin (N-[[(4S)-Hexahydro-1-methyl-2,6-dioxo-4-pyrimidinyl]carbonyl]-L-histidyl-L-prolinamid), das die folgende Struktur aufweist: 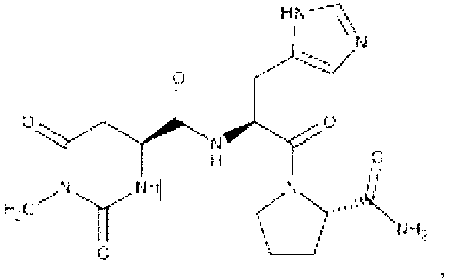 zur Verwendung bei der Behandlung eines Leidens, das mit Zell- oder Gewebeseneszenz oder -entzündung in Verbindung steht, wobei das Leiden Arthrose ist.

## Revendications

1. Taltiréline, (N-[[(4S)-hexahydro-1-méthyl-2,6-dioxo-4-pyrimidinyl]carbonyl]-L-histidyl-L-prolinamide), répondant à la structure : 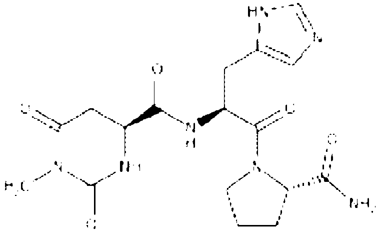 pour une utilisation destinée au traitement d'une condition associée à une sénescence ou une inflammation cellulaire ou tissulaire, la condition étant l'arthrose.
